# EUROPEAN PATENT APPLICATION

(11) **EP 2 664 274 A1**
(43) Date of publication of application: **20.11.2013**
(21) Application number: 13167537.3
(22) Date of filing: 13.05.2013
(51) Int. Cl.: A61B 5/00

(54) **Pathogen detection systems and methods**

(30) Priority: 16.05.2012 US 201261647753 P; 18.10.2012 US 201213654649
(71) Applicant: Hill-Rom Services, Inc., Batesville, IN 47006-9167 (US)
(72) Inventor: Ribble, David, Indianapolis, IN 46202 (US); McCleerey, Michelle, Raleigh, NC 27604 (US); Agdeppa, Eric, Cincinnati, OH 45249 (US); Lawrence, Brian, Cincinnati, OH 45242 (US); Penninger, Jason, Indianapolis, IN 46220 (US)
(74) Representative: Findlay, Alice Rosemary

(57) **Abstract**

Pathogen and chemical detection systems for use with a patient support apparatus and related methods are disclosed. A fluid or biologic sample from the vicinity of a person supported by a person support apparatus is acquired and transmitted to a pathogen detection sensor. A graphical caregiver interface is supplied output from the pathogen detection sensor and detects the presence of a chemical. The graphical caregiver interface communicates with an electronic medical records database, and alarm and a nurse call system.

## Description

Detection of pathogens and odors in the vicinity of a patient supported by a person support apparatus is a continuing challenge. Secretions of chemicals and / or biomarkers by a patient may be indicative of presence of pathogens, the rate of change in development of certain conditions and / or onset of other conditions. While several systems exist to detect and monitor pathogens a need exists to continue development in this area.

The present disclosure includes one or more of the following features alone or in any combination.

One embodiment of a system for detecting pathogens for use with a person support apparatus comprises a system for detecting pathogens for use with a person support apparatus, comprising a fluid transport system which receives fluid transported away from the vicinity of a person supported by a person support apparatus, a processor, at least one pathogen detection sensor, said at least one pathogen sensor configured to detect a pathogen in fluid transported in said fluid transport system to determine presence of a pathogen in a quantity exceeding a predetermined threshold and to communicate with said processor.

An embodiment of a system for detecting an odor in the vicinity of a hospital bed comprises at least one odor sensor to which fluid from the vicinity of a person supported by a hospital bed is transported. The at least one odor sensor configured to communicate with a processor which may be a graphical caregiver interface.

An embodiment of a system for purification of air in the vicinity of a person supported by a person support apparatus comprises at least one chemical sensor configured to sense at least one chemical in fluid transported away from a person supported by the person support apparatus. The chemical sensor is configured to communicate with a processor which may be a graphical caregiver interface and the graphical caregiver interface is also configured to communicate with an air filtration system.

One embodiment of a system for monitoring wound development comprises a person support apparatus and at least one chemical sensor configured to sense at least one chemical in fluid transported away from a person supported by the person support apparatus. The chemical sensor is configured to communicate with a graphical caregiver interface, the graphical caregiver interface comprising a memory configured to store signals supplied by the chemical sensor for a predetermined time.

There is disclosed a method for detecting a chemical in the vicinity of a person support apparatus comprises the operation of transporting fluid away from a person supported by a person support apparatus, testing the fluid transported from a person supported by the person support apparatus and reporting result of said testing to a graphical caregiver interface. The method comprises a further operation of determining whether said result indicates presence of at least one chemical by determining whether quantity of the at least one chemical detected is greater than a predetermined threshold. An alarm is activated to alert a caregiver when quantity

The invention will now be further described by way of example with reference to the accompanying drawings, in which:

FIG. 1 is a block diagram of a system for detecting pathogens for use with a person support apparatus wherein a portion of the fluid transport system comprises a mattress topper, constructed according to one or more of the principles disclosed herein;

FIG. 2 is a block diagram of another system for detecting pathogens for use with a person support apparatus wherein at least a portion of the pathogen sensor is housed within a mattress topper, constructed according to one or more of the principles disclosed herein;

FIG. 3 is a block diagram of another system for detecting pathogens for use with a person support apparatus wherein a pathogen containment screen is configured to envelope at least a portion of a patient supported by a person support apparatus, constructed according to one or more of the principles disclosed herein;

FIG. 4 is a block diagram of another system for detecting pathogens for use with a person support apparatus wherein at electric potential is used to generate fluid flow in a fluid transport system, constructed according to one or more of the principles disclosed herein;

FIG. 5 is a block diagram of a system for detecting pathogens for use with a person support apparatus wherein the pathogen sensing system is a free standing device, constructed according to one or more of the principles disclosed herein;

FIG. 6 is a block diagram of another system for detecting pathogens for use with a person support apparatus wherein the pathogen sensing system 26 is housed within the air filtration system, constructed according to one or more of the principles disclosed herein;

Various features and advantageous details are explained more fully with reference to the non-limiting embodiments and examples that are described and/or illustrated in the accompanying drawings and detailed in the following description. It should be noted that the features illustrated in the drawings are not necessarily drawn to scale, and features of one embodiment may be employed with other embodiments as the skilled artisan would recognize, even if not explicitly stated herein. Descriptions of well-known components and processing techniques may be briefly mentioned or omitted so as to not unnecessarily obscure the embodiments described. The examples used herein are intended merely to facilitate an understanding of ways in which the embodiments may be practiced and to further enable those of skill in the art to practice the embodiments described herein. Moreover, it is noted that like reference numerals represent similar parts throughout the several views of the drawings.

It is understood that the embodiments are not limited to the particular methodology, protocols, devices, apparatus, materials, applications, etc., described herein, as these may vary. It is also to be understood that the terminology used herein is used for the purpose of describing particular embodiments only.

Unless defined otherwise, all technical and scientific terms used herein have the same meanings as commonly understood by one of ordinary skill in the art.

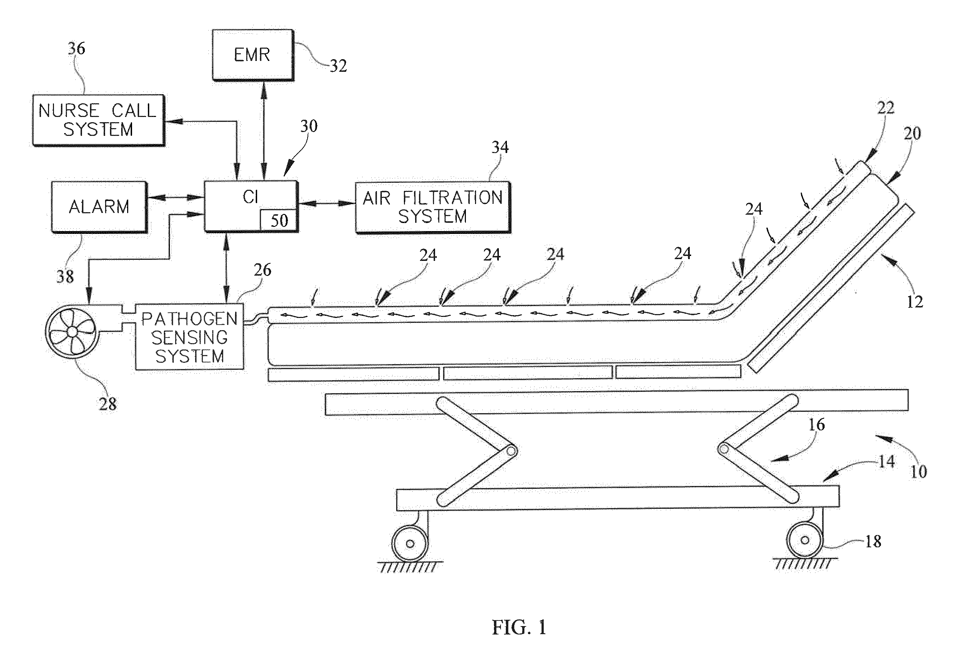

One embodiment of a system to detect a pathogen in the vicinity of a person support apparatus 10 is shown in FIG. 1. In this embodiment, the person support apparatus 10 is a bed, however, in other embodiments the person support apparatus 10 may be a wheelchair, stretcher or any other apparatus configured to support a person thereon. The person support apparatus 10 in this embodiment comprises an upper frame 12 which is supported over a lower frame 14 by supports 10. The upper frame 12 comprises one of more sections and the support 16 is configured to variably elevate at least one section of the upper frame 12 with respect to the lower frame 14. The lower frame 14 rests on at least one caster wheel 18 in this embodiment, allowing the person supported apparatus 10 to be transported. A person support surface 20 rests on the person support apparatus 10. In this embodiment the person support surface 20 is a mattress and comprises fluid filled bladders, in other embodiments the person support surface may be made of any combination of bladders, foam and other polymeric materials. A mattress topper 22 is configured to be positioned on top of the person support surface 20 such that a person can be supported on top of the mattress topper 22. The mattress topper 22 comprises fluid permeable pores 24 on its surface which is configured to support a person. Fluid permeable pores 24 are configured to allow air, water and microbial particles to pass through them. A pump 28 is configured to suck fluid in through the fluid permeable pores 24 and into a pathogen sensing system 26 through a fluidic connection as shown in FIG. 1. The pump 28 is configured to perform one or more functions of the person support surface 20 including but not limited to inflation of bladders. In another embodiment the pump 28 is dedicated for use by the system to detect pathogens. In this embodiment the pathogen sensing system 26 comprises electrodes whose surfaces are polymerized. The constitution of the surroundings affect the electrical resistance measured between the electrodes and this change in resistance is used to identify the constitution of the surroundings by comparison with known resistance and / or change in resistance values. In other embodiments, any other type of sensors may be used including but not limited to metal oxide type semi-conductor (MOS) sensors and surface acoustic wave devices. In another embodiment, protein based biosensors such as odorant binding proteins (OBPs), sensory appendage proteins (SAPs), odorant or gustatory receptors, serpentine receptors and / or odorant degrading enzymes (ODEs) may be used along with electrochemical transducers to generate an electrical signal in response to detection of a chemical. The pump 28 and the pathogen sensing system 26 are configured to communicate with a controller which may be or form part of a graphical caregiver interface (GCI) 30. In this embodiment, the GCI 30 comprises a memory 50 to store information supplied by the pathogen sensing system 26 for a predetermined time. Memory 50 may be of any type including volatile and non-volatile. In this embodiment the GCI 30 is mounted on the person support apparatus 10 and controls the function of one or more features of the person support apparatus 10. In another embodiment the system comprises a dedicated GCI 30. The GCI 30 is configured to communicate with a nurse call system 36 configured to alert a caregiver. The GCI 30 in this embodiment is also configured to activate an alarm 38. Alarm 38 is an audio alert in this embodiment, in other embodiments alarm 38 may be any combination of audio, visual and / or tactile alerting system. The GCI 30 is also configured to communicate with an electronic medical records (EMR) database 32. In this embodiment the GCI 30 is configured to communicate with an air filtration system 34. The GCI 30 is configured to control the air filtration system 34 based on the results provided by the pathogen sensing system 26. In one prophetic example if a high concentration of sweat is detected in the data transmitted by the pathogen sensing system 26 to the GCI 30, the partial pressure of water vapor and / or temperature and / or volume of air being supplied to the room housing are modified to induce an environment to optimize sweat production. In another prophetic example, the air filtration system 34 comprises a controllable valve so as to prevent any air from leaking out of the room containing a patient without first cleansing the air if a determination is made that the data from the pathogen sensing system 26 indicates presence of a certain pathogen above a predetermined threshold. The air filtration system 34 may rely on any combination of ionization, electrostatic and mechanical filtration technologies.

During operation of the system shown in FIG. 1 the GCI 30 is configured to control the operation of the pump 28 to create a suction pressure allowing suction of fluids through the fluid permeable pores 24 and into the pathogen sensing system 26. In this embodiment, the pathogen sensing system 26 communicates with the GCI 30 and supplies to the GCI 30 results of the testing on the fluid flowing into the pathogen sensing system 26. The GCI 30 in this embodiment allows a caregiver to set thresholds for presence of one or more chemicals. The thresholds may be set in the form of limits or a rate of change. In another embodiment, the caregiver may program detection of specific combinations of chemicals as a threshold. These thresholds are stored in the memory 50 in this embodiment. When results obtained from the pathogen sensing system 26 indicate that a predetermined threshold has been exceeded the GCI 30 activates alarm 38 in this embodiment. The GCI 30 also communicates with a nurse call system 36 when the predetermined threshold has been exceeded. In this embodiment the GCI 30 logs the results of testing provided by the pathogen sensing system 26 in an EMR 32 database, while in another embodiment the GCI 30 obtains information from the EMR 32 which is used to determine the thresholds. The thresholds may be so selected that presence of any chemical or combinations of chemicals may be sought for detection. In this embodiment chemical signatures indicative of disease causing micro-organisms and / or vectors are sensed by the pathogen sensing system 26. In another embodiment results supplied by the pathogen sensing system 26 are evaluated by the graphical caregiver interface 30 to seek chemical signatures indicative of biochemical markers of wound development such as IL-1a and Creatine Phosphokinase (CPK).

Any suitable controller may be employed to perform the functions described above and below for the GCI 30 and it will be appreciated that the controller need not necessarily be or form part of a graphical caregiver interface.

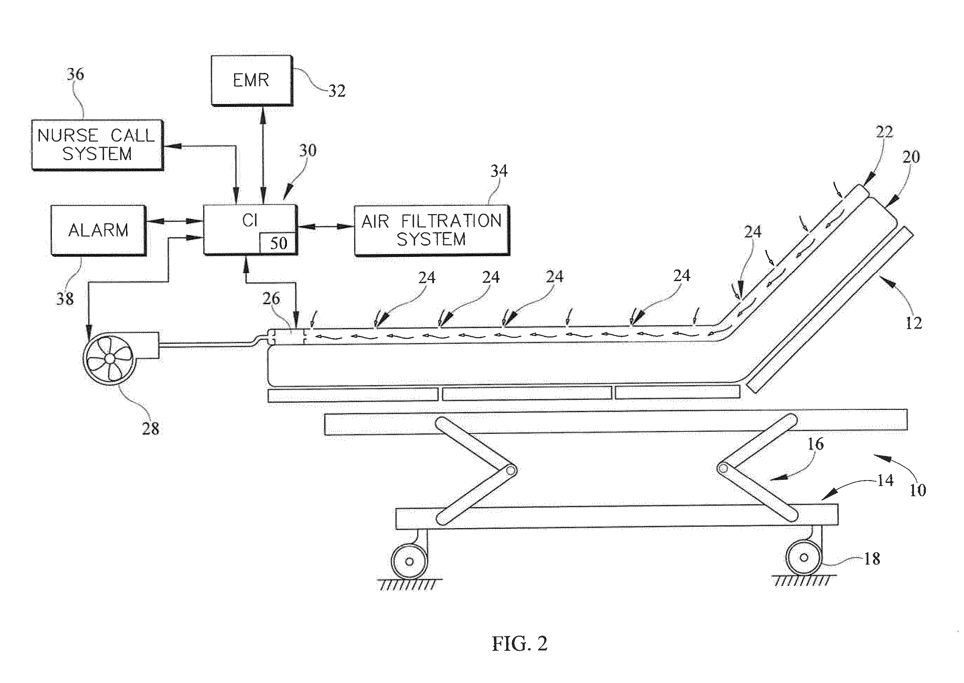

In the embodiment of a system to detect a pathogen in the vicinity of a person support apparatus 10 shown in FIG. 2, the pathogen sensing system 26 is housed within the mattress topper 22. In another embodiment the pump 22 is also housed within the mattress topper 22. The person support surface 20 in another embodiment comprises fluid permeable pores 24 and provides the fluidic path between the fluid permeable pores 24 and the pathogen sensing system 26.

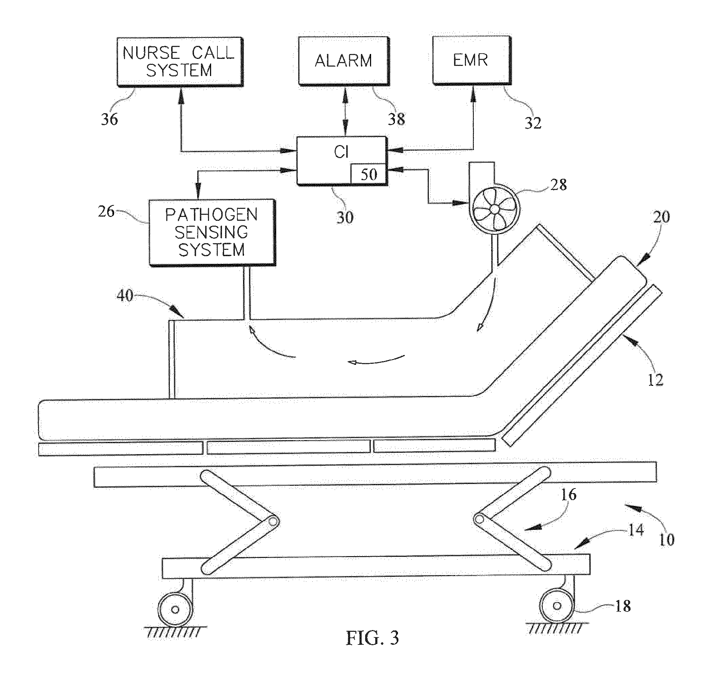

In FIG. 3 another embodiment of a system to detect a pathogen in the vicinity of a person is depicted. In the system shown in FIG. 3, a pathogen containment screen 40 is placed over the person support surface 20. The pathogen containment screen is made of a substantially fluid impermeable material and is configured to fit over at least a portion of a person supported by the person support apparatus 20. The pump 28 in controlled by GCI 30 and supplies air with a positive pressure within the volume of air contained by the pathogen containment screen 40. The pathogen sensing system 26 is in fluidic communication with the volume of air contained by the pathogen containment screen 40. The pathogen sensing system 26 is configured to communicate with a GCI 30 which comprises a memory 50. The GCI 30 is also configured to communicate with a nurse call system 36, an alarm 38 and an EMR 32 in this embodiment.

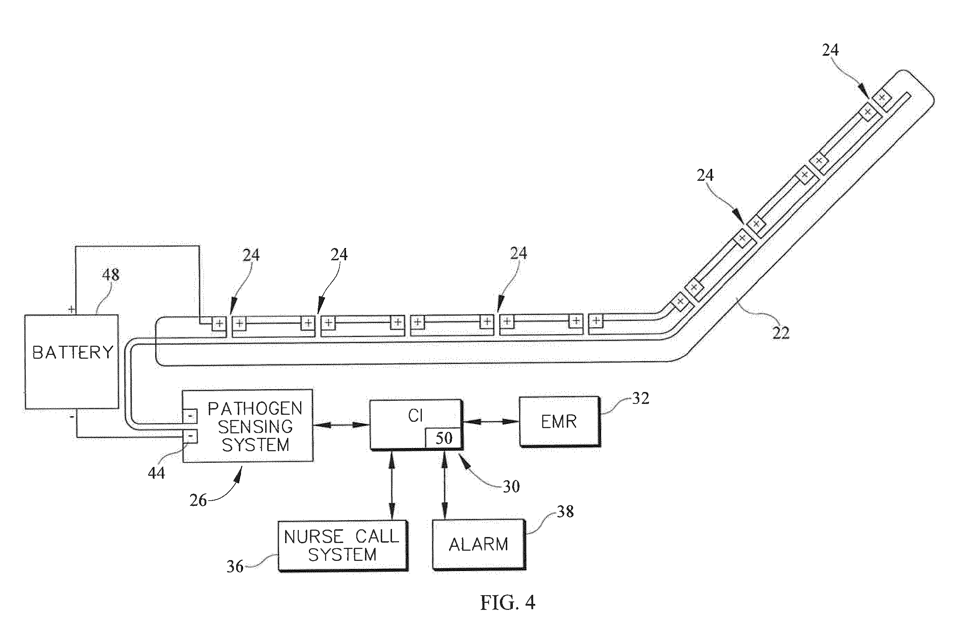

In the embodiment of a system to detect a pathogen in the vicinity of a person support apparatus 10 shown in FIG. 4 an electrical potential is used to induce flow of fluid between fluid permeable pores 24 and the pathogen sensing system 26. FIG. 4 shows a mattress topper 22 which comprises positively charged electrical leads 42 attached substantially close to the inlet of the fluid permeable pores 24 in this embodiment. Negatively charged electrical leads 44 are attached to the pathogen sensing system 26. The positively charged electrical leads 42 and negatively charged electrical leads 44 are attached to the positive and negative terminals of a battery 48 respectively. The battery 48 and electrical leads form a differential in electrical potential between the fluid permeable pores 24 and the pathogen sensing system 26. Particulate, volatile and liquid matters are drawn through the fluidic connection between the fluid permeable pores 24 and the pathogen sensing system 26 based on their electrophoretic mobility. In one embodiment the GCI 30 provides electrical potential between the positively charged electrical leads 42 and the negatively charged electrical leads 44 instead of utilization of a battery 48. The pathogen sensing system 26 is in communication with a GCI 30 comprising a memory 50. The GCI 30 is configured to communicate with an EMR 32, nurse call system 36 and an alarm 38 in this embodiment. In another embodiment the fluid permeable pores 24, positively charged electrical leads 42 and the fluidic path to the pathogen sensing system 26 are incorporated in a person support surface 20.

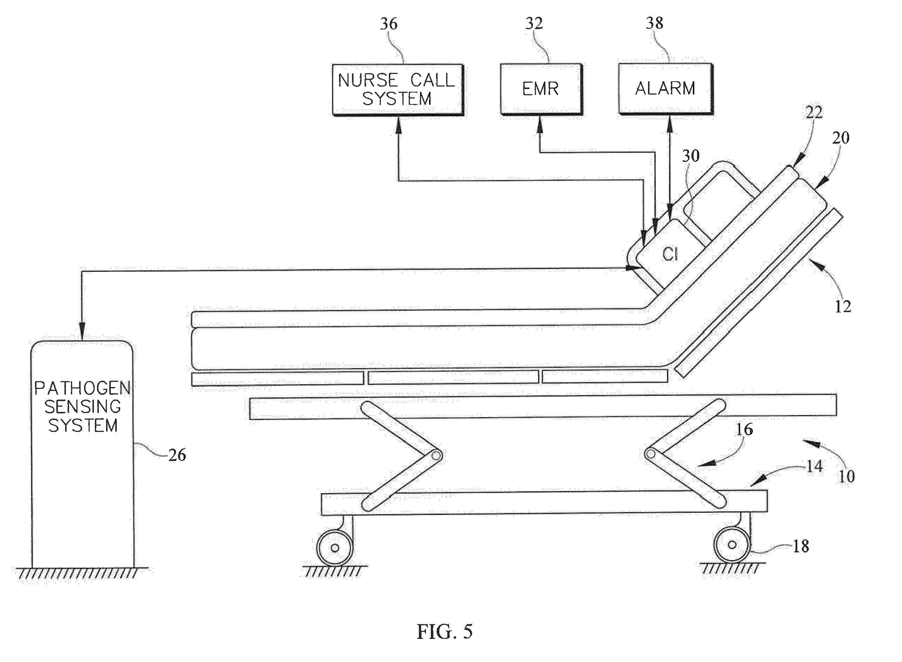

In the embodiment shown in FIG. 5, a standalone pathogen sensing system 26 samples air circulating around a person support apparatus and is configured to communicate the sensed data to a GCI 30 of a person support apparatus 10.The stand alone pathogen sensing system 26 may be free standing structure resting on the floor, be mounted on the person support apparatus 10 or be supported by any other structure in the vicinity of the person support apparatus 10. GCI 30 is configured to communicate with a nurse call system 36, EMR 32 and an alarm 38.

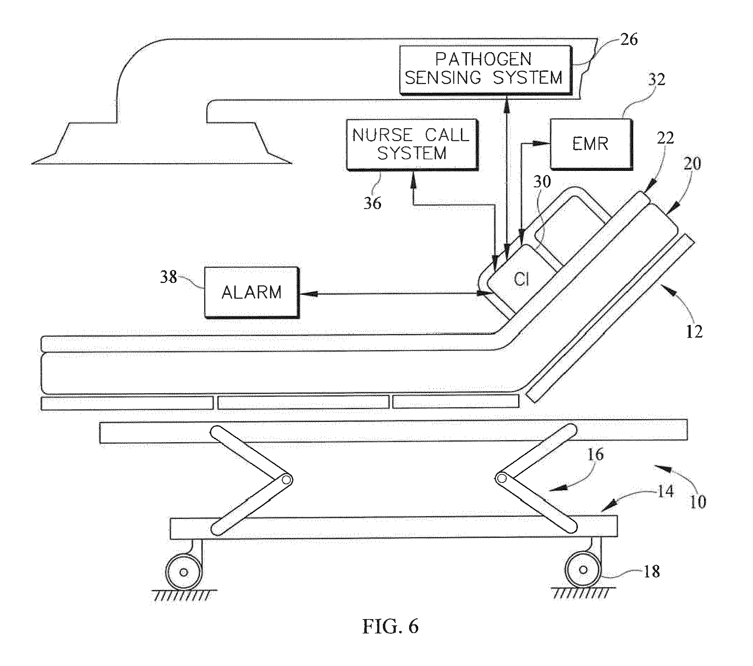

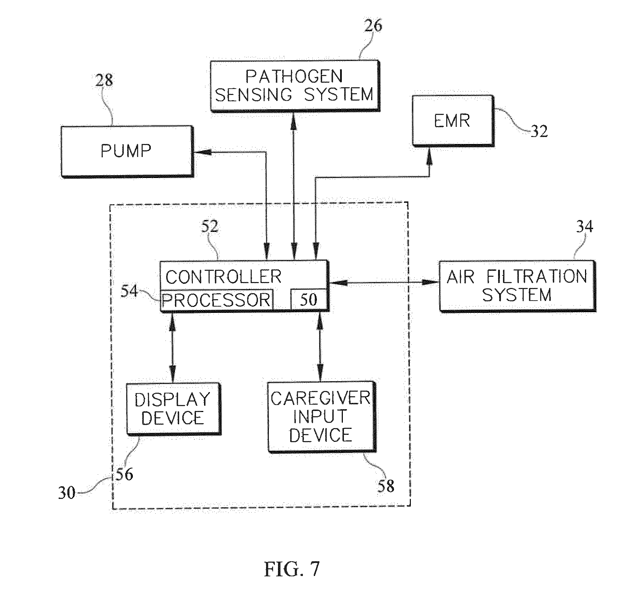

In another embodiment shown in FIG. 6, the pathogen sensing system 26 is housed within the air filtration system 34. Air filtration system 34 is the heating, ventilation and air conditioning (HVAC) system of the room which houses the person support apparatus 10. The pathogen sensing system 26 samples air flowing through the air filtration system 34 and is configured to communicate the sensed data to a GCI 30 of a person support apparatus 10. The GCI 30 is configured to control the air filtration system 34 based on the results provided by the pathogen sensing system 26. In one prophetic example if a high concentration of sweat is detected in the data transmitted by the pathogen sensing system 26 to the GCI 30, the partial pressure of water vapor and / or temperature and / or volume of air being supplied to the room housing are modified to induce an environment to optimize sweat production. In another prophetic example, the air filtration system 34 comprises a controllable valve so as to prevent any air from leaking out of the room containing a patient without first cleansing the air if a determination is made that the data from the pathogen sensing system 26 indicates presence of a certain pathogen above a predetermined threshold. GCI 30 is configured to communicate with a nurse call system 36, EMR 32 and an alarm 38.

Preferred embodiments are described herein. Of course, variations of those preferred embodiments will become apparent to those of ordinary skill in the art upon reading the foregoing description. The inventor expects skilled artisans to employ such variations as appropriate. Moreover, any combination of the above-described elements in all possible variations thereof is encompassed unless otherwise indicated herein or otherwise clearly contradicted by context.

The disclosures of any references and publications cited above are expressly incorporated by reference in their entireties to the same extent as if each were incorporated by reference individually.

Embodiments of the invention can be described with reference to the following numbered clauses, with preferred features laid out in the dependent clauses:
1. A system for detecting pathogens for use with a person support apparatus, comprising:
   a fluid transport system configured to transport fluid away from skin of a person supported by a person support apparatus;
   at least one pathogen detection sensor, said at least one pathogen sensor configured to detect a pathogen in fluid transported in said fluid transport system;
   a graphical caregiver interface, said pathogen detection sensor configured to communicate with said graphical caregiver interface which is configured to determine presence of a pathogen in a quantity exceeding a predetermined threshold, said graphical caregiver interface configured to control at least one function of a person support apparatus.
2. The system of clause 1 wherein said fluid transport system comprises a pump to displace fluid.
3. The system of clause 2 wherein said pump is an electro-osmosis type pump.
4. The system of clause 2, wherein said pump uses positive pressure to transport fluid away from a person's skin.
5. The system of clause 1, wherein said at least one pathogen detection sensor is a metal oxide semi-conductor type sensor.
6. The system of clause 1, wherein said at least one pathogen detection sensor is an organic semi-conducting polymer type sensor.
7. The system of clause 1, wherein said at least one pathogen detection sensor is a surface acoustic wave type sensor.
8. The system of clause 1, wherein said at least one pathogen detection sensor is a protein based sensor.
9. The system of clause 1, wherein a portion of said fluid transport is integrated into a mattress topper.
10. The system of clause 1, wherein said graphical caregiver interface is configured to communicate with an electronic medical record database.
11. The system of clause 1, wherein said graphical caregiver interface is configured to communicate with a nurse call system.
12. The system of clause 1, wherein said graphical caregiver interface is configured to communicate with an alarm.
13. The system of clause 1, wherein said graphical caregiver interface is configured to communicate with an air filtration system.
14. A system for detecting an odor in the vicinity of a hospital bed, comprising:
   at least one odor sensor;
   a fluid transport system configured to transport fluid away from skin of a person supported by a hospital bed to said at least one odor sensor;
   a graphical caregiver interface, said at least one odor sensor configured to communicate with said graphical caregiver interface.
15. The system of clause 14 wherein said fluid transport system comprises a pump to displace fluid.
16. The system of clause 15 wherein said pump is an electro-osmosis type pump.
17. The system of clause 15, wherein said pump uses positive pressure to transport fluid away from a person's skin.
18. The system of clause 14, wherein said at least one odor sensor is a metal oxide semi-conductor type sensor.
19. The system of clause 14, wherein said at least one odor sensor is an organic semi-conducting polymer type sensor.
20. The system of clause 14, wherein said at least one odor sensor is a surface acoustic wave type sensor.
21. The system of clause 14, wherein said at least one odor sensor is a protein based sensor.
22. The system of clause 14, wherein a portion of said fluid transport system is integrated into a mattress topper.
23. The system of clause 14, wherein said graphical caregiver interface is configured to communicate with an electronic medical record database.
24. The system of clause 14, wherein said graphical caregiver interface is configured to communicate with a nurse call system.
25. The system of clause 14, wherein said graphical caregiver interface is configured to communicate with an alarm.
26. A system for purification of air in the vicinity of a person supported by a person support apparatus, comprising:
   a person support apparatus;
   a fluid transport system configured to transport fluid away from a person supported by the person support apparatus;
   at least one chemical sensor configured to sense at least one chemical in fluid transported away from a person supported by the person support apparatus;
   a graphical caregiver interface, said at least one chemical sensor configured to communicate with said graphical caregiver interface;
   an air filtration system, said graphical caregiver interface configured to communicate with said air filtration system.
27. The system of clause 26 wherein said a portion of said fluid transport system is integrated into a mattress topper.
28. A system for monitoring wound development, comprising:
   a person support apparatus;
   a fluid transport system configured to transport fluid away from a person supported by said person support apparatus;
   at least one chemical sensor configured to sense at least one chemical in fluid transported away from a person supported by said person support apparatus;
   a graphical caregiver interface, said at least one chemical sensor configured to communicate with said graphical caregiver interface, said graphical caregiver interface comprising a memory configured to store signals supplied by the at least one chemical sensor for a predetermined time.
29. The system of clause 28 wherein said graphical caregiver interface is configured to compare a signal supplied by the at least one sensor with an earlier signal supplied by said at least one chemical sensor and alert a caregiver if said comparison indicates a change greater than a predetermined threshold.
30. A method for detecting a chemical in the vicinity of a person support apparatus comprising:
   transporting fluid away from a person supported by a person support apparatus;
   testing said fluid transported from a person supported by said person support apparatus;
   reporting result of said testing to a graphical caregiver interface.
   determining whether said result indicates presence of at least one chemical based on whether quantity of said at least one chemical detected is greater than a predetermined threshold;
   activating an alarm to alert a caregiver when quantity of said at least one chemical detected is greater than a predetermined threshold.
31. The method of clause 30 further comprising the operation of determining if said at least one chemical is an indicator of presence of a pathogen.

## Claims

1. A system for detecting pathogens for use with a person support apparatus, comprising:
a fluid transport system which receives fluid transported away from the vicinity of a person supported by a person support apparatus;
a processor;
at least one pathogen detection sensor, said at least one pathogen sensor configured to detect a pathogen in fluid transported in said fluid transport system to determine presence of a pathogen in a quantity exceeding a predetermined threshold and to communicate with said processor.

2. The system for detecting pathogens of claim 1 wherein the fluid transport system is configured to transport fluid away from the vicinity of the person.

3. The system for detecting pathogens of either claim 1 or claim 2 wherein the processor is configured to control at least one function of the person support apparatus.

4. The system for detecting pathogens of any preceding claim wherein the threshold is a limit.

5. The system for detecting pathogens of any one of claims 1 to 3 wherein the threshold is a rate of change.

6. The system for detecting pathogens of any preceding claim, wherein said processor is configured to communicate with an electronic medical record database.

7. The system for detecting pathogens of any preceding claim, wherein said processor is configured to communicate with a nurse call system.

8. The system for detecting pathogens of any preceding cliam, wherein said processor is configured to communicate with an alarm.

9. The system for detecting pathogens of any preceding claim wherein the fluid transport system is bounded in part by a pathogen containment screen.

10. The system for detecting pathogens of any preceding claim including a person support surface and a pathogen containment screen configured to fit over at least a portion of the person support surface and wherein the pathogen detection sensor is in fluid communication with a volume of air contained by the pathogen containment screen and the person support surface.

11. The system for detecting pathogens of any one of claims 1 to 8 comprising:
a person support surface with pores therein and wherein the pathogen detection sensor is part of a pathogen sensing system, the system for detecting pathogens also comprising means for establishing a difference in electric potential between the pores and the pathogen sensing system.

12. The system for detecting pathogens of any one of claims 1 to 8 wherein the fluid transport system includes a facility HVAC system and wherein the pathogen sensing system is housed within the HVAC system.

13. The system for detecting pathogens of any one of claims 1 to 8 wherein said fluid transport system comprises a pump to displace fluid.

14. The system for detecting pathogens of claim 13, wherein said pump uses positive pressure to transport fluid away from a person's skin.

15. The system for detecting pathogens of any one of claims 1 to 8, wherein the pathogen sensing system is integrated into a person support surface.
